# EUROPÄISCHE PATENTANMELDUNG

(11) **EP 1 837 049 A1**
(43) Veröffentlichungstag der Anmeldung: **26.09.2007**
(21) Anmeldenummer: 07101954.1
(22) Anmeldetag: 08.02.2007
(51) Int. Cl.: A61N 5/01, A61N 5/10

(54) **Partikeltherapie-Anlage und Verfahren zum Ausgleichen einer axialen Abweichung in der Position eines Partikelstrahls**

(30) Priorität: 20.03.2006 DE 102006012680
(71) Anmelder: SIEMENS AKTIENGESELLSCHAFT, 80333 München (DE)
(72) Erfinder: Kaiser, Werner, 84085 Langquaid (DE); Rohdjess, Heiko Dr., 91091 Grossenseebach (DE); Lazarev, Vitali Dr., 91341 Röttenbach (DE)

(57) **Zusammenfassung**

Die Partikeltherapie-Anlage (2), umfasst eine drehbare Gantry (4) mit einem im Betrieb erzeugbaren Partikelstrahl (20) und eine Messvorrichtung (6, 6a) zum Bestimmen einer Position des Partikelstrahls (20). Zur Korrektur einer Abweichung der Position des Partikelstrahls (20) von einer axialen Sollposition ist die Gantry (4) in Axialrichtung (Y) verfahrbar.

## Beschreibung

Partikeltherapie-Anlage und Verfahren zum Ausgleichen einer axialen Abweichung in der Position eines Partikelstrahls einer Partikeltherapie-Anlage

Die Erfindung betrifft eine Partikeltherapie-Anlage, umfassend eine drehbare Gantry mit einem im Betrieb erzeugbaren Partikelstrahl. Die Erfindung betrifft weiterhin ein Verfahren zum Ausgleichen einer axialen Abweichung in der Position eines Partikelstrahls einer Partikeltherapie-Anlage mit einer drehbaren Gantry.

Die Strahlentherapie, insbesondere die Partikeltherapie mittels Protonen oder Schwerionen für die Behandlung von Krebserkrankungen, gewinnt heutzutage mehr und mehr an Bedeutung. Eine Partikeltherapie wird insbesondere bei Patienten angewandt, bei denen die herkömmliche Röntgenbestrahlung nicht ausreichend genutzt werden kann, weil der Tumor entweder zu tief im Körper sitzt oder aber von empfindlichen Organen umgeben ist. Eine Partikeltherapie wird teilweise mit Hilfe einer drehbaren Gantry durchgeführt. Die Gantry umschließt einen Bestrahlungsraum in den ein Patiententisch hineingefahren wird.

Für eine möglichst präzise Bestrahlung muss das zu bestrahlende Gewebe des Patienten möglichst genau im Isozentrum (der Treffpunkt des Strahls bei der Rotation der Gantry) der Anlage positioniert werden. Die Zielgenauigkeit des Strahls wird neben der Patientenpositionierung und anderen Faktoren auch von der geometrischen Präzision der Gantry maßgeblich beeinflusst. Dabei treten durch thermische Dehnungen der Gantry, durch Lagerfehler oder Verformungen aufgrund der Gewichtskraft Abweichungen des Isozentrums von seiner Sollposition auf, in der das zu bestrahlende Gewebe plaziert ist.

In einem zylindrischen Koordinatensystem können diese Abweichungen mittels einer Rotationsachse, einer Radialrichtung und einer Winkellage der Gantry beschrieben werden. Die Abweichung in Radialrichtung ist für die Bestrahlung unkritisch, weil dadurch der Strahlweg durch Luft verlängert oder verkürzt wird. Eine Veränderung des Strahlwegs in der Luft hat einen unerheblichen Einfluss auf die Eindringtiefe des Strahls im Patienten. Die Eindringtiefe hängt primär von der Strahlenergie ab, deshalb können die Abweichungen in der Radialrichtung vernachlässigt werden. Die Abweichungen entlang der Rotationsachse und in der Winkellage der Gantry wirken sich allerdings auf die genaue Führung des Strahls nachteilig aus und müssen im Betrieb der Gantry korrigiert werden.

Ein Verfahren zur Bestimmung der Form, der Größe und des Ortes des geometrischen Zentrums eines mechanischen Isozentrums von Radiotherapiebehandlungseinheiten mit rotierbarer Gantry sowie ein Aufbau einer Protonen-Gantry sind in "Isocenter characteristics of a external ring proton gantry", Int. J. Radiat. Oncol. Biol. Phys. 60 (2004), S. 1622-1630, von M.F. Moyers und W. Lesyna beschrieben.

Die US 4,112,306 A beschreibt den Aufbau eines Neutronentherapie-Geräts, das über eine Gantry zum Verkippen eines Zyklotrons verfügt, in dem die zur Neutronenerzeugung nötigen Protonen beschleunigt werden. Dabei verlassen die Neutronen das Zyklotron über einen Kollimator, dessen Querschnitt variierbar ist.

In der DE 102 41 178 A1 ist eine Gantry-Anordnung zur isokinetischen Führung eines Teilchenstrahls beschrieben, die Magnete aufweist, die den von einem Teilchenbeschleuniger axial eingeschossenen Teilchenstrahl umlenken. Die Gantry-Anordnung umfasst eine rotationssysmetrische Primärstruktur, deren Steifigkeit derart bemessen ist, dass die vertikalen Verschiebungen der Magnete aufgrund ihres Gewichts in sämtlichen Richtungen gleich groß (isokinetisch) sind. Hierfür werden die Magnete auf Kreisbahnen um eine theoretische Drehachse bewegt, die gegenüber einer horizontalen Längsachse der Gantry-Anordnung in unbelasteten Zustand verschoben ist. Als Bestrahlungszielpunkt ist hierbei der Schnittpunkt zwischen dem Teilchenstrahler und der lastverschobenen theoretischen Drehachse definiert. Zur Lagerung der Primärstruktur sind an deren Enden zwei Auflagertragringe vorgesehen, die mit stationären Lagerständern zusammenwirken. Dabei ist einer der stationären Lagerständer als Loslager und der andere als Festlager ausgestaltet.

Der Erfindung liegt die Aufgabe zugrunde, eine Partikeltherapie-Anlage anzugeben, deren Aufbau eine Kompensation der Abweichung ihres Isozentrums ermöglicht. Der Erfindung liegt weiterhin die Aufgabe zugrunde, ein Verfahren zum Ausgleichen von einer Abweichung in der Position eines Isozentrums einer Partikeltherapie-Anlage anzugeben, welches eine besonders hohe Zielgenauigkeit des Partikelstrahls ermöglicht.

Die erstgenannte Aufgabe wird erfindungsgemäß gelöst durch eine Partikeltherapie-Anlage, umfassend eine drehbare Gantry mit einem im Betrieb erzeugbaren Partikelstrahl und eine Messvorrichtung zum Bestimmen einer Position des Partikelstrahls, wobei die Gantry zur Korrektur einer Abweichung der Position des Partikelstrahls von einer axialen Sollposition in Axialrichtung verfahrbar ist.

Die Erfindung basiert auf der Überlegung, dass eine sehr hohe Präzision bei der Partikelbestrahlung eines Tumors auch bei entstehenden Abweichungen in der Position des Isozentrums, z.B. aufgrund thermischen Dehnung der Gantry, erreicht wird, indem die Position des Partikelstrahls insbesondere während der Bestrahlung bestimmt und korrigiert wird. Zum Beginn der Bestrahlung wird die Lage des Isozentrums der Gantry erfasst und das zu bestrahlende Gewebe wird im Isozentrum positioniert. Diese Ausgangsposition des Isozentrums, in der sich während der Bestrahlung der Tumor befindet, definiert eine Sollposition des Isozentrums. Wenn eine axiale Abweichung der Position des Partikelstrahls ermittelt wird, die zu einer Abweichung der Lage des Isozentrums führt, wird die Gantry in Axialrichtung, d.h. entlang ihrer Rotationsachse verfahren, um diese Abweichung zu korrigieren.

Der wesentliche Vorteil dieser Partikeltherapie-Anlage ist, dass durch ihre Ausgestaltung eine ausgesprochen hohe Zielgenauigkeit des Partikelstrahls gewährleistet ist. Insbesondere ist bei der vorliegenden Anlage ein Ausgleich der Abweichung des Isozentrums bzw. des Partikelstrahls in der Größenordnung von 0,1 mm erzielbar. Diese Genauigkeit übertrifft um das Mehrfache die Genauigkeit der bekannten Partikeltherapie-Anlagen.

Hierbei kann die Bestimmung der Position des Partikelstrahls auf mehrere Weisen erfolgen. Einerseits kann eine Messvorrichtung eingesetzt werden, die während der Bestrahlung kontinuierlich oder wiederholt die aktuelle Position des Partikelstrahls erfasst. Alternativ ist es auch möglich, dass eine Reihe von Kalibriermessungen durchgeführt wird, mit deren Hilfe eine Relation beispielweise zwischen den Gantryparametern, der Umgebungstemperatur, der Position eines strahlbestimmenden Elementes, wie etwa der letzte Magnet in Richtung der Strahlführung und der Position des Partikelstrahls aufgestellt wird. Dadurch kann bei der Bestrahlung des Patienten die Position des strahlbestimmenden Elements mittels der Messvorrichtung gemessen und die Position des Partikelstrahls unter Berücksichtigung der Meßreihe ermittelt werden.

Das Verfahren der Gantry bei detektierten Abweichungen wird gemäß einer bevorzugten Ausführungsform konstruktiv realisiert, indem zur Lagerung der Gantry ein Loslager an einem vorderen Gehäuseteil im Bereich eines Strahlaustritts und ein verschiebbares Festlager an einem hinteren Gehäuseteil vorgesehen sind. Die Gantry umfasst in der Regel ein mindestens zweiteiliges zylinderförmiges Gehäuse, wobei die unterschiedlichen Gehäuseteile unterschiedlich groß sind. Mit ihrem vorderen Gehäuseteil umschließt die Gantry einen Bestrahlungsraum, in den ein Patiententisch hineingefahren wird. Am anderen Ende der Gantry erfolgt der Strahleintritt und das hintere Gehäuseteil weist einen Durchmesser auf, der etwa um das Dreifache kleiner ist als der Durchmesser des vorderen Gehäuseteils. Dementsprechend muss ein Lager, das am vorderen Gehäuseteils angeordnet ist, deutlich größere Lasten aufnehmen als ein Lager, das am hinteren Gehäuseteil vorgesehen ist. Durch die Verwendung von einem Loslager im Bereich des vorderen Gehäuseteils ist eine besonders kostengünstige Konstruktion der Anlage erreicht. Das Loslager nimmt ausschließlich Radialkräfte auf und die Gantry kann bei ihrer Dehnung im Bereich der Loslagerung "wandern". Diese Versetzung der Gantry bezogen auf das feststehende Loslager wird durch die Bestimmung der Position des Partikelstrahls erfasst. Entsprechend erfolgt die Korrektur der Position des Partikelstrahls durch die Verschiebung des Festlagers. Das Festlager, das sowohl Radial- als auch Axialkräfte aufnimmt, ist fest mit der Gantry verbunden und seine relative Position gegenüber der Gantry ändert sich nicht. Somit wird das Festlager samt der Gantry verfahren, um die Abweichungen der Position des Partikelstrahls auszugleichen.

Bevorzugt umfasst die Messvorrichtung ein optisches Wegmesssystem, mit dem insbesondere die Position des Partikelstrahls direkt gemessen wird. Berührungslose optische Meßsysteme zeichnen sich durch ihre Verschleißfestigkeit und hohe Auflösung aus, die bei etwa 0,005% bis 0,1% vom Meßbereich liegt. Dadurch wird eine vorzügliche Genauigkeit bei der Bestimmung der Position des Partikelstrahls bzw. bei der Korrektur der Abweichung erreicht. Die Position des Partikelstrahls kann beispielweise durch die Einbringung eines Films in den Strahlgang und die Durchführung einer geometrischen Auswertung des vom Partikelstrahl gebildeten Flecks gemessen werden. Die Auswertung des Strahlflecks kann z.B. auf einem Leuchtschirm durch eine CCD-Kamera erfolgen.

Um die Reibung und den Widerstand beim Verfahren der Gantry herabzusetzen, ist das Loslager zweckdienlicherweise als ein hydrostatisches Radiallager ausgebildet. Hydrostatische Lager sind durch einen Schmierstoffkreislauf gekennzeichnet, wodurch ein beinah verschleißfreier Betrieb gewährleistet ist.

Nach einer bevorzugten Ausgestaltung ist eine Führung für das Festlager vorgesehen. Eine Führung weist lediglich einen Translationsfreiheitsgrad auf, wodurch eine vorgegebene Zwangsbewegung des Festlagers erzielt ist. Somit wird gewährleistet, dass die Gantry lediglich in Axialrichtung verfahren wird. Die Führung kann beispielweise eine Schiene, eine Wellenführung oder eine Rollenführung sein.

Um eine mögliche Verschiebung des Partikelstrahls durch ein Abkommen des Festlagers von seiner korrigierten Stellung zu vermeiden, ist bevorzugt ein Arretierungselement zur Arretierung des Festlagers vorgesehen. Als Arretierungselement kann z.B. ein Sicherungsbolzen, eine Schraube und eine Klemmvorrichtung eingesetzt werden.

Die zweitgenannte Aufgabe wird erfindungsgemäß gelöst durch ein Verfahren zum Ausgleichen einer axialen Abweichung in der Position eines Partikelstrahls einer Partikeltherapie-Anlage mit einer drehbaren Gantry, wobei eine Position des Partikelstrahls bestimmt wird und bei einer Abweichung dieser Position von einer axialen Sollposition die Gantry derart in Axialrichtung verfahren wird, dass die Abweichung korrigiert wird.

Die im Hinblick auf die Partikeltherapie-Anlage ausgeführten Vorteile und bevorzugten Ausführungsformen sind sinngemäß auf das Verfahren zu übertragen.

Bevorzugt ist die Gantry über ein feststehendes Loslager und ein verschiebbares Festlager derart gelagert, dass beim Verfahren der Gantry zur Korrektur der Abweichung das Festlager verschoben wird. Weiterhin bevorzugt wird die Position des Partikelstrahls optisch gemessen.

Die Abweichung der Partikelstrahlposition z.B. durch mechanische Verformungen oder thermische Dehnungen der Gantry spielt sich sehr langsam ab. Deshalb wird gemäß einer vorteilhaften Variante die Abweichung in der Position des Partikelstrahls in regelmäßigen Zeitabständen gemessen und korrigiert. Insbesondere wird die Position des Partikelstrahls während der Bestrahlung etwa alle 30 min ermittelt. Dadurch wird gewährleistet, dass die Bestrahlung des Patienten nicht unnötig oft durch die Messungen unterbrochen wird.

Zweckdienlicherweise wird das Festlager in seiner korrigierten Stellung arretiert.

Weiterhin vorteilhaft ist, dass zum Ausgleich der Abweichung einer Winkellage des Partikelstrahls die Gantry rotiert wird. Hierbei ist die Korrektur der abgewichenen Winkellage auf die einfachste Weise realisiert, bei der die Repositionierung des Partikelstrahls durch den Antrieb der Gantry erfolgt.

Ausführungsbeispiele der Erfindung werden im Folgenden anhand einer Zeichnung näher erläutert. Hierin zeigen schematisch:
- FIG 1: einen Schnitt in Axialrichtung durch eine Partikeltherapie-Anlage die eine Gantry und eine Messvorrichtung umfasst, und
- FIG 2: eine Vordersicht auf die Gantry gemäß FIG 2.

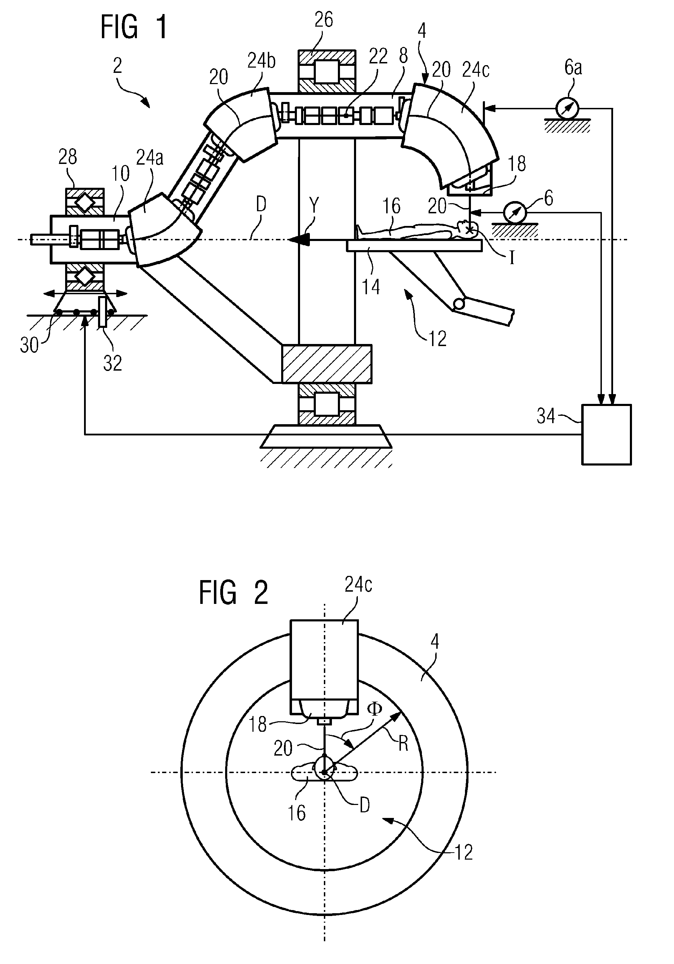

In FIG 1 ist eine Partikeltherapie-Anlage 2 gezeigt, die im Wesentlichen eine drehbare Gantry 4 und eine Messvorrichtung 6, 6a umfasst. Die Axialrichtung der Gantry 4, die auch mit ihrer Drehachse D übereinstimmt, ist mit Y bezeichnet. In Axialrichtung Y weist die Gantry 4 ein vorderes und ein hinteres Gehäuseteil 8, 10 auf. Das vordere Gehäuseteil 8 umschließt einen Bestrahlungsraum 12, in dem ein Patiententisch 14 mit einem darauf liegenden Patienten 16 hineingefahren wird. Aus einer Wand des Bestrahlungsraums 12 ragt ein Austrittsfenster 18 heraus, auch als Nozzle bezeichnet, aus dem ein Partikelstrahl 20, insbesondere ein Protonenstrahl austritt. Der Patient 16 ist derart positioniert, dass sein zu bestrahlendes Gewebe im Isozentrum I der Gantry 4 liegt. Somit wird eine Sollposition des Isozentrums I bzw. des Partikelstrahls 20 festgelegt, von der das Isozentrum I bzw. der Partikelstrahl 20 während der Bestrahlung nicht abweichen darf, um mögliche Schäden in dem den Tumor umgebenden Gewebe zu vermeiden.

Das hintere Gehäuseteil 10 weist einen deutlich kleineren Durchmesser als das vordere Gehäuseteil 8 auf. Im Bereich des hinteren Gehäuses 10 tritt der Partikelstrahl 20 von einem hier nicht dargestellten Teilchenbeschleuniger in die Gantry 4 ein und wird über eine Strahlenführung 22, die zum Umlenkung des Partikelstrahls 20 Magnete 24a, 24b, 24c umfasst, in Richtung der Nozzle 18 geführt.

Die Gantry 4 ist mittels zweier Lager 26, 28 drehbar gelagert. Am vorderen Gehäuseteil 8 ist ein Loslager 26 angeordnet, das fest steht. Dieses Loslager 26 nimmt nur Radialkräfte, d.h. Kräfte senkrecht zur Axialrichtung Y, auf und ist insbesondere nach Art eines hydrostatischen Radiallagers ausgebildet. Am hinteren Gehäuseteil 10 ist ein Festlager 28 angeordnet, das sowohl Radial- als auch Axialkräfte aufnimmt. Das Festlager 28 kann über eine Führung 30 axial verschoben werden, was durch einen Doppelpfeil angedeutet ist. Wenn das Festlager 28 sich in einer gewünschten Stellung befindet, wird es in dieser Stellung mittels eines Arretierelements 32 festgehalten.

Mit einer derartigen Lagerung der Gantry 4 ist die Möglichkeit gegeben, bei thermischen Dehnungen oder Verformungen der Gantry 4 oder bei Lagerfehlern, die zu einer Abweichung des Partikelstrahls 20 bzw. des Isozentrums I von der Sollposition führen, die Gantry 4 derart zu verfahren, dass die Abweichungen korrigiert werden. Für diesen Zweck wird die Position des Partikelstrahls 20 im Bestrahlungsraum 12 mit Hilfe der Messvorrichtung 6 und/oder der Messvorrichtung 6a bestimmt. Die Messvorrichtung 6 ist in diesem Ausführungsbeispiel ein berührungsloses optisches Wegmesssystem, welches direkt die Lage des Partikelstrahls 20 ermittelt. Bei thermischen Dehnungen der Gantry 4 verschiebt sich die Gantry 4 bezogen auf das feststehende Loslager 26 in entgegengesetzter Richtung des Pfeils Y. Diese Verschiebung der Gantry 4, die allerdings sehr langsam geschieht, zieht nach sich eine Verschiebung des Partikelstrahls 20. Deshalb wird während der Bestrahlung des Patienten 16 in regelmäßigen Zeitabständen, beispielweise in Zeitabständen von etwa 30 min, überprüft, ob eine Abweichung der Position des Partikelstrahls 20 vorliegt, die zu einer Verschiebung des Isozentrums I führt.

Eine Steuereinheit 34 ist mit der Messvorrichtung 6 und der Führung 30 verbunden. Die Steuereinheit 34 wertet die Signale der Messvorrichtung 6 aus und im Falle einer Abweichung des Isozentrums I von seiner Sollposition steuert sie die Führung 30 derart an, dass diese Abweichung durch ein Verfahren der Gantry 4 ausgeglichen wird. Anschließend wird das Festlager 28 in seiner korrigierten Stellung arretiert.

Alternativ oder ergänzend zur Messvorrichtung 6, die direkt die Position des Partikelstrahls 20 misst, kann eine Messvorrichtung 6a vorgesehen sein, die ebenfalls nach Art eines optischen Wegmesssystems ausgebildet ist, jedoch zum Bestimmen der Position eines Elements der Strahlenführung 22 dient, insbesondere des letzten Magnets 24c vor der Nozzle 18. In diesem Fall werden vorangehende Kalibriermessungen verwendet, welche die Position und Ausrichtung des Partikelstrahls 20 in Abhängigkeit von der Position des Magnets 24a, den Gantryparametern und der Umgebungstemperatur geben.

Eine Vordersicht auf die Gantry 4 ist in FIG 2 dargestellt. In dieser Figur ist eine Radialrichtung R der Gantry 4 gezeigt. Weiterhin gezeigt ist ein Winkel Φ um den die Nozzle 18 verfahrbar ist, um den Tumor aus einer anderen Winkellage zu bestrahlen. Die Radialrichtung R und der Winkel Φ geben neben der Axialrichtung Y die Achsen eines zylindrischen Koordinatensystems an, entlang deren sich der Partikelstrahl 20 bei thermischen Dehnungen oder mechanischen Verformungen der Gantry 4 verschieben kann.

Die Abweichungen des Partikelstrahls 20 in Radialrichtung R werden nicht berücksichtigt, da ihr Einfluß auf die Eindringtiefe des Partikelstrahls 20 im Körper des Patienten 16 unwesentlich ist. Die Eindringtiefe des Partikelstrahls 20 hängt hauptsächlich von der Energie des Strahls 20 ab und ein etwas längerer oder kürzerer Strahlweg in der Luft hat im wesentlichen keine Auswirkung auf die Strahlenergie.

Eine Abweichung der Winkellage der Nozzle 18 wird korrigiert, indem die Gantry 4 um ihre Drehachse D, die in dieser Figur nur als ein Punkt angedeutet ist, gedreht wird, bis der Partikelstrahl 20 bzw. das Isozentrum I sich wieder in seiner Sollposition befindet. Dies erfolgt über den eigenen Antrieb der Gantry 4, der ebenfalls von der in FIG 1 dargestellten Steuereinheit 34 angesteuert wird.

## Patentansprüche

1. Partikeltherapie-Anlage (2), umfassend eine drehbare Gantry (4) mit einem im Betrieb erzeugbaren Partikelstrahl (20) und eine Messvorrichtung (6, 6a) zum Bestimmen einer Position des Partikelstrahls (20), wobei die Gantry (4) zur Korrektur einer Abweichung der Position des Partikelstrahls (20) von einer axialen Sollposition in Axialrichtung (Y) verfahrbar ist.

2. Partikeltherapie-Anlage (2) nach Anspruch 1,
bei der zur Lagerung der Gantry (4) ein Loslager (26) an einem vorderen Gehäuseteil (8) im Bereich eines Strahlaustritts (18) und ein verschiebbares Festlager (28) an einem hinteren Gehäuseteil (10) vorgesehen sind.

3. Partikeltherapie-Anlage (2) nach Anspruch 1 oder 2,
bei der die Messvorrichtung (6, 6a) ein optisches Wegmesssystem umfasst.

4. Partikeltherapie-Anlage (2) nach Anspruch 2 oder 3,
bei der das Loslager (26) ein hydrostatisches Radiallager ist.

5. Partikeltherapie-Anlage (2) nach einem der Ansprüche 2 bis 4,
bei der eine Führung (30) für das Festlager (28) vorgesehen ist.

6. Partikeltherapie-Anlage (2) nach einem der Ansprüche 2 bis 5,
bei der ein Arretierelement (32) zur Arretierung des Festlagers (28) vorgesehen ist.

7. Verfahren zum Ausgleichen einer axialen Abweichung in der Position eines Partikelstrahls (20) einer Partikeltherapie-Anlage (2) mit einer drehbaren Gantry (4), wobei eine Position des Partikelstrahls (20) bestimmt wird
und bei einer Abweichung dieser Position von einer axialen Sollposition die Gantry (4) derart in Axialrichtung (Y) verfahren wird, dass die Abweichung korrigiert wird.

8. Verfahren nach Anspruch 7,
bei dem die Gantry (4) über ein feststehendes Loslager (26) und ein verschiebbares Festlager (28) derart gelagert ist, dass beim Verfahren der Gantry (4) zur Korrektur der Abweichung das Festlager (28) verschoben wird.

9. Verfahren nach Anspruch 7 oder 8,
bei dem die Position des Partikelstrahls (20) optisch gemessen wird.

10. Verfahren nach einem der Ansprüche 7 bis 9,
wobei die Abweichung in der Position des Partikelstrahls (20) in regelmäßigen Zeitabständen gemessen und korrigiert wird.

11. Verfahren nach einem der Ansprüche 8 bis 10
wobei das Festlager (28) in seiner korrigierten Stellung arretiert wird.

12. Verfahren nach einem der vorhergehenden Ansprüche,
wobei zum Ausgleich der Abweichung einer Winkellage des Partikelstrahls (20) die Gantry (4) rotiert wird.
